# EUROPEAN PATENT SPECIFICATION

(11) **EP 0 873 682 B1**
(45) Date of publication and mention of the grant of the patent: **04.09.2002**
(21) Application number: 98107918.9
(22) Date of filing: 18.06.1991
(51) Int. Cl.: A01H 4/00

(54) **Improved zea mays (L.) genotypes with capability of long term, highly efficient plant regeneration**
Verbesserte Genotypen von Zea mays (L.) mit der Veranlagung zur langfristigen, höchst effizienten Pflanzenregeneration
Génotypes améliorés de Zea mays (L.) avec capacité de régénération en plantes de haute efficacité de longue durée

(30) Priority: 23.06.1990 EP 90111945
(43) Date of publication of application: 28.10.1998
(62) Divisional of application: 91109933.1
(73) Proprietor: Aventis CropScience GmbH, 65929 Frankfurt am Main (DE)
(72) Inventor: Dudits, Denes, Dr., 6726 Szeged (HU); Morocz, Sandor, Dr., 6727 Szeged (HU); Nemeth, Janos, Dr., 6727 Szeged (HU); Donn, Günter, Dr., 65619 Hofheim (DE)

(56) References cited:
- No relevant documents disclosed

## Description

Despite the fact that several alternative approaches such as microinjection into the cells, macroinjection or bombardment with DNA coated particles of the intact or cultured tissues have been claimed as methods for genetic transformation of crop plants, according to the available experimental data protoplasts are ideal and the most widely used objects for parasexual genetic manipulation methods including DNA transformation and somatic hybridization. These genetic manipulations require a methodology for protoplast isolation and culture. For practical applications obtaining fertile regenerants from the manipulated protoplasts is a basic prerequisite. Among the monocotyledonous crop species maize is one of the most studied tissue cultures. Several publications have shown that the potential for plant regeneration from various cultured organs including anthers and immature embryoids is highly genotype-dependent (Green and Phillips, Crop Sci. 15, 417 (1975), Morocz, Tag-Ber., Acad. Landwirtsch.-Wiss. DDR, Berlin (1983), Duncan et al., Planta 165, 322 (1985)). Improvements of culture conditions can only partially overcome these limitations (Duncan et al., Planta 165, 322 (1985)).

Over recent years there has been much research into the development of genotypes and processes to get the problem of plant regeneration under control. EP 29 24 435 describes a process in which fertile plants can be obtained starting from a relatively non-mucilaginous, friable and granular maize callus. Shillito et al. (Bio/Technology 7, 581 (1989)) and Prioli et al. Bio/Technology 7, 589 (1989)) describe the regeneration of plants from maize protoplasts. However, in these cases regenerable protoplasts could be isolated only after a long in vitro culture period of 7 to 8 or 20 to 40 months. Furthermore, the regenerated plants show abnormalities in their morphology and their fertility.

It has now been found that a new Zea mays (L.) genotype can produce tissue and cell suspension cultures with the reproducible capacity to regenerate stably plants which are normal and predominantly fully fertile.

The invention thus relates to
- A genotype of Zea mays (L.) which is auxin-autotrophic and from which protoplasts, which reproducibly and stably regenerate into normal fertile plants, can be produced, as well as the regenerated plants and parts and progeny of those plants;
- a protoplast produced from the genotype as characterized under 1. as well as cells regenerated from this protoplast, said cells having the capability to regenerate a fertile plant.

The cell line DSM 6009 is a preferred representative of the genotype as characterized above and was deposited at the "Deutsche Sammlung von Mikroorganismen und Zellkulturen GmbH" on June 1, 1990 under the conditions of the Budapest Treaty with the above-cited registration number.

The present invention, therefore, also relates to cell line DSM 6009.

The genotypes as characterized above and the techniques of culture initiation and maintenance, the suspension culture establishment, the protoplast isolation and culture techniques were developed in the following steps:

### Step (a): Evaluation of a wide range of commonly used genotypes for tissue culture potential

Maize inbreds, stocks and land races are tested for their ability to form callus cultures from anthers and somatic organs such as immature embryos, apical and lateral meristems. For anther culture, anthers with mononucleate microspores are placed onto anther culture initiation medium and are incubated in the dark preferably at 21 to 28°C for 40 to 90 days.

For somatic cultures the inocula (e.g. 1 to 3 mm size immature embryos, 0.5 to 0.7 cm size meristems) either intact or sliced are placed on somatic culture initiation and maintenance medium in the light at 21 to 28°C for 30 to 60 days. The resulting cultures are tested for their ability to regenerate on plant regeneration medium. The genotypes responding with regenerable tissue cultures are used for cross-hybridization, and the hybrid embryos are tested in the same way with additional scoring for their regenerability followed by at least one year of maintenance in tissue culture. The material derived from anther culture is maintained on haploid-maintenance medium. The hybrids of regenerable inbreds, stocks or land races usually produce embryogenic cultures with increased frequency. They do not lose the ability to regenerate after the first few passages and are more easily regenerated to plants from their tissue cultures than their parents.

### Step (b): Production of starting synthetics from the genotypes selected in step (a)

Several genotypes from cultured anthers and somatic inocula that proved to be regenerable in step (a) are crossed through successive generations to obtain synthetics for anther and somatic tissue cultures, each with the involvement of 6 to 8 genotypes.

### Step (c): Selection for improved recombinants with superior tissue culture properties

Anthers and immature embryos or meristems from synthetics as described under step (b) are used as inocula to obtain improved regenerable embryogenic cultures. Out of several hundreds of regenerable cultures a special type of culture is obtained which can be maintained as an early-embryogenic, abundantly proliferating callus. After one year of maintenance, genotypes like H 229 and OK 281, the parent genotypes of DSM 6009, are obtained by selecting and keeping only those cultures which produce normally developing regenerants from calli grown both in surface and in suspension culture. The isolated protoplasts of these selected fertile, regenerable, early embryogenic cultures again show variation in their viability and culturability. Genotypes with positive responses in protoplast experiments are preferably kept. After the selection process, regenerants from the first cycle genotypes are cross-hybridized to obtain second cycle genotypes, such as DSM 6009 (HE 89), that are equally well-suited for the initiation of regenerable haploid or somatic cell and tissue cultures, and produce totipotent protoplasts from appropriate cultures.

### Step (d): Optimization of the conditions for regulation of the developmental stages of the genotype in the selected cultures

Frequent and regular passages (2 to 3 times at 1 to 2 weeks' intervals) of the H 229, OK 281 and DSM 6009 tissue cultures on maintenance media suppress further differentiation of the early embryoids and cause redifferentiations of the later developmental stages to the early stages. Once the homogeneous early-embryogenic culture is obtained, it can easily be maintained on different culture media at less frequent passage intervals (e.g. 1 month). With infrequent passages or preferably on transfer to plant regeneration medium late-embryogenic cultures develop from the early embryogenic cultures. The late-embryogenic cultures consist of asynchronously germinating embryoids which, after a second transfer onto plant regeneration medium, yield a large number of regenerated plants. The early-embryogenic cultures of these genotypes are characterized by their high flexibility in respect of culture media. Commonly used culture media, such as MS (Murashige/Skoog, Physiol. Plant 15, 473 (1962)), B5, SH or N6 (Chu C.C. et al., Sci. Sin. 16, 659 (1975)) can all be used to initiate or maintain H 229, OK 281 and DSM 6009 tissue cultures. The N6M medium is preferred because it is possible to maintain these cultures without the loss of capability for fertile plant regeneration for several years on this medium (e.g. for 4 years in the case of H 229). The close interaction between the high-level expression of the embryogenic traits in these genotypes and the optimal composition of the N6M medium for the manifestation of the regenerable, embryogenic culture formation is demonstrated by the applicability of N6M medium without hormones not only for plant regeneration but also for initiation and maintenance of embryogenic cultures from these genotypes. As a consequence, an important characteristic of these genotypes is their auxin-autotrophic growth capability.

### Step (e): Improvement of maintenance of the suspension cultures which allow the regeneration of fertile plants after prolonged periods of culture

Due to the fact that fast growing cell populations have the tendency to accumulate cellular mutants there was a need to improve the liquid culture medium in order to extend the time during which a suspension culture can be regenerated into phenotypically normal and fully fertile plants. Unexpectedly, it was found that this can be achieved by replacement of N6-micronutrients by MS-micronutrients and by decreasing, compared to surface cultures, the 2,4-dichlorophenoxyacetic acid (2,4-D) content and increasing sucrose (the latter to about 3 %) in the medium. This so-called N6M medium was also suitable for the friable early embryogenic callus culture of DSM 6009. After about 2 passages in this medium the suspension could be used to isolate in good yield regenerable protoplasts from this genotype.

### Step (f): Protoplast isolation and culture

Protoplasts from the genotypes described can be produced by incubation with lysing enzymes, such as cellulases and pectinases. The enzymes can also be used as mixtures, the concentration of a single enzyme being 0.05 to 4 % (weight per volume). The optimal composition of the aqueous incubation solution as well as enzyme concentration can be established for each type of tissue in simple tests. For osmotic stabilization of protoplasts the incubation solution should contain osmotic substances such as mannitol, sorbitol, glucose, sucrose, fructose, KNO₃ or other typical salts of plant nutrient media. The osmolarity of the enzyme solution is preferably in the range from 500 to 750 mOsm, in particular in the range from 600 to 700 mOsm. The pH can vary in the range from 4.5 to 6.0. Preferably the pH is in the range from 5.5 to 6.0 and is stabilized by addition of 1 to 75 mM phosphate and 1 to 10 mM morpholinoethanesulfonic acid (MES).

Depending on the enzyme concentration, protoplasts are produced after 3 to 20 hours. During incubation careful suspension by shaking is preferred, in particular on an orbital shaker at 10 to 50 rpm.

After incubation the protoplasts can be transferred to a growth medium containing salts, vitamins, amino acids, a synthetic auxin, like 2,4-D or dicamba, as well as sugars, preferably a mixture of fructose, glucose and sucrose.

Most preferably the protoplasts are cultured in thin layers (e.g. 0.1 - 1 mm) of ppN6M protoplast culture medium in sterile glass or plastic culture vessels, sealed properly to prevent evaporation from the protoplast cultures, in dim light at temperatures between 21 to 26°C. The density of protoplasts necessary for cell and colony formation is between 5-7×10⁵ and 3-5×10⁶ protoplasts per ml of protoplast culture medium. After a culture period of 20 to 40 days colonies visible to the unaided eye develop in the protoplast culture medium. The period necessary for the formation of visible colonies may be shortened by the stepwise addition of small amounts of suspension or protoplast culture medium (e.g. 0.2 to 0.5 ml at 3- to 4-day intervals) three weeks after the start of the protoplast culture. The visible colonies are placed on maintenance or regeneration medium or alternatively may be used to start a new suspension culture.

### Step (g): Plant regeneration and growth of the regenerated plants to maturity from protoplast-derived cultures

The protoplast-derived colonies are placed on plant regeneration medium, where they develop into mature somatic embryos which eventually germinate into plantlets. On hormone-free media the relatively homogeneous early embryogenic cultures form on their surface well developed embryogenic tissue clusters. When the mature and germinating embryoids are placed on fresh hormone-free plant regeneration medium a large number of healthy plants differentiate. When the plantlets have a size of 5 to 7 cm and several roots at the first nodes, they are removed from the culture vessel and separated from each other and from the adhering agar pieces. Their root system is freed from weak non-nodal roots and culture medium using tap water. The plantlet is then transplanted into soil. An appropriate soil is an equal part mixture of peat, sand and perlite or any commercially available horticulture soil. The plants are grown under nylon bags or in a humid atmosphere for ten days, illuminated constantly for two days and periodically afterwards with an intensity to provide normal green coloration and vigorous growth of the plants. The plants are watered and nourished with fertilizers regularly to ensure their normal growth and development. Only the carefully cultivated plants that retain the green color of their first leaves are used to produce seeds via self-or cross-pollination. Most of the plants that are transplanted into soil before ageing starts in the culture vessels and which are cultivated properly produce well-developed plants devoid of abnormalities in the reproductive organs.

### Step (h): Strain identification and genotype protection with the aid of RFLP (restriction fragment length polymorphism)

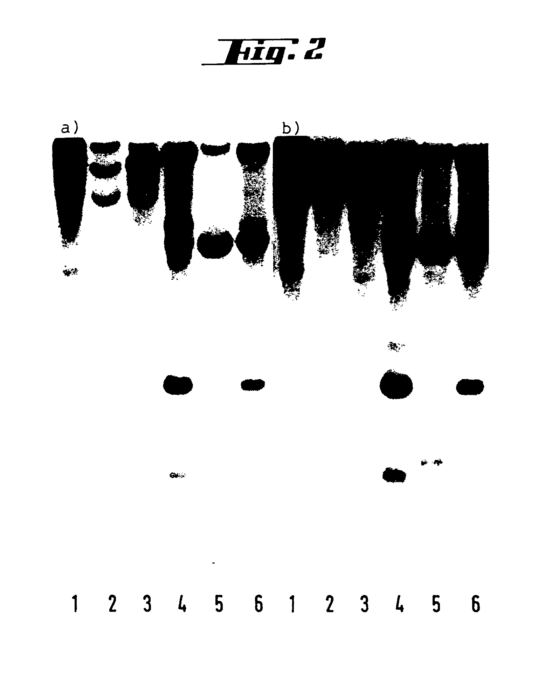

In addition to the typical morphological and physiological traits as they become visible in tissue culture initiation and maintenance, plant regeneration and plant stability characteristics of the selected genotypes and the distribution of hybridizing restriction fragments after the use of defined molecular probes allow the identification of the described genotypes. DNA is isolated from the parental lines (H 229 and OK 281) and the resulting highly embryogenic hybrid DSM 6009. As an example we present the hybridization patterns after digestion of this plant DNA with HindIII and BamHI restriction enzymes by using a cDNA clone for a putative auxin receptor gene cloned from maize (U. Tillman et al. The EMBO J. 1988, 2463-2467). As can be seen in fig. 2 all of the analyzed genotypes exhibit a unique and characteristic pattern of hybridizing bands.

Fig. 2a, b show restriction fragment polymorphism visible after hybridizing with a ³²P-labeled cDNA insert for a putative auxin receptor gene (U. Tillmann et al. The EMBO J. 1988, 2463-2467).

| | | |
|---|---|---|
| 1 | OK | |
| 2 | H 229 | Hind III |
| 3 | DSM 6009 | |
| 4 | OK | |
| 5 | H 229 | Bam HI |
| 6 | DSM 6009 | |

The exposure time of figure 2b was approximately 3 times longer than of figure 2a.

### Example 1:

### Production of the opaque somatic culture synthetic MR Syn So₂o₂/87 and the predominantly early-embryogenic culture OK281

Plants from different maize genotypes were grown in the field. Three to four plants were self-pollinated and the fertilized ears were harvested 12-14 days after pollination. After removal of husks and silks the ears were surface sterilized in 0.15 % sodium hypochlorite solution containing 0.5 % household detergent for 10 minutes followed by rinsing 3 times with sterile deionized water. Ten embryos per ear were then aseptically dissected and placed with their flat plumule and radicle side down in contact with the surface of 50 ml solidified (0.8 % agar) somatic culture initiation and maintenance medium in 10 × 2 cm Petri dishes. The embryo cultures were incubated for 30-60 days under illumination at 25 ± 2°C. After evaluation, the embryogenic cultures were transferred to fresh medium at monthly intervals and tested for plant regeneration capacity on plant regeneration medium. From the tested genotypes the inbreds A188, GK3/H and W64A, the genetic stock Mangelsdorf's Tester (MT) and a white flint local variety (WFLV) were found to form regenerable cultures under the described conditions. These genotypes, however, formed predominantly late-embryogenic cultures, often referred to as type I culture in the maize tissue culture literature. Their regenerative potential gradually decreased upon successive transfers to fresh medium and after 6 months' maintenance only occasional small regenerable sectors were present. When hybrids of two regenerable genotypes were produced and their embryo cultures were tested in the same way an improvement in the propagation rate and regenerability was observed. The culture from the A188 × W64A hybrid, for example, was maintained for 3 years without the loss of regenerative potential. To facilitate further combination of the possible advantageous genes from the original genotypes a multiple-generation crossing program was started. Two opaque (o₂o₂) sources which were either non-regenerable under these conditions (Oh 43o₂o₂) or not tested for their ability to regenerate from tissue cultures (H Temp Ao₂o₂) were included. To combine the opaque gene with the genes responsible for the regenerable trait, first the opaque source genotypes had to be crossed with the regenerable genotypes. Therefore these genotypes were planted, cultivated and their ears were isolated before silk emergence. The isolated ears of the regenerable genotypes were cross-pollinated with pollen from genotypes carrying the opaque gene. Thus the single cross hybrids (SC1-SC10) were obtained by crossing A188, GK3/H, W64A, MT, and WFLV with Oh43o₂o₂ and H Temp Ao₂o₂.

The kernels of these 10 different single crosses were harvested, planted, grown and isolated as before for the second series of cross-pollinations to obtain the double cross (DC1-DC5) hybrid kernels from SC1 × SC7, SC2 × SC8, SC3 × SC9, SC4 × SC10 and SC5 × SC6 crosses, respectively. From the first and second pollination series only one harvested ear was taken from each cross-pollination for further generation. From the harvested DC kernels homozygous o₂o₂ kernels were selected on the basis of their phenotype and were planted. At the flowering time in a third pollination series each DC hybrid (DC1 to DC5) was crossed with all the others to provide 4 harvested ears from each of the 20 cross-combinations. From the resulting 80 ears 10 kernels per ear were bulked, planted and randomly sibpollinated to give a possibility for recombination of the desired genes. The hundred best ears were taken from the sibbed, harvested plant material, 10 kernels from each ear were bulked and regarded as a starting somatic culture synthetic (MR Syn So₂o₂/87). The obtained synthetic was planted and the flowering plants were self-pollinated. From each of 100 self-pollinated ears 10 immature embryos were plated in the described manner. About 30 percent of the cultured embryos (286 out of 950) produced regenerable cultures, the majority of them being the late-embryogenic type. From the spontaneous early-embryogenic cultures obtained, often referred to as type II or friable embryogenic culture in the relevant literature, the OK281 culture was selected on the basis of its vigorous growth, lack of necrosis, easy regenerability into plants and fertility of regenerated plants.

### Example 2:

### Production of the anther culture synthetic MR Syn A/85 and the early-embryogenic haploid culture H 229

Different maize genotypes were planted and cultivated to the appropriate physiological stage to collect tassels with the uppermost leaves uncovered. The outer leaves were unfolded and discarded. The remaining tassels still in the covering whorl were put into plastic bags and cold treated at a temperature of 4 to 8°C in the refrigerator for 10-30 days. After this treatment the covering leaves were removed and the tassel sectors with anthers carrying late mononucleate microspores were selected using microscopic examination of anthers squashed in 2 % aqueous orcein solution. The florets carrying the desired microspores were surface sterilized in 0.1 % sodium hypochlorite solution containing 0.5 % household detergent for 10 minutes followed by rinsing the florets in deionized sterile water 3 × for 10 minutes. From the surface sterilized florets about 100 anthers were dissected and placed on 50 ml anther culture initiation medium in 10 × 2 cm glass Petri dishes. The plated anthers were incubated in the dark for 40-90 days at 25°C. Two local varieties, the Golden Glow (GG) and a white dent local variety (WDLV), produced at low percentage (below 1 %) haploid calli from the cultured anthers. None of them regenerated plants on the regeneration medium. The two local varieties (GG and WDLV) were crossed, hybrid kernels were planted and anthers of the resulting F1 plants were tested in the same way. From the obtained haploid embryoids one culture could be used for multiple plant regeneration during a period of one year before it lost its regenerative potential. To combine the embryoid formation potential, the plant regeneration capacity and plant characters necessary for the survival and growth of the regenerants transplanted into the soil, ten plants from the hybrid of the two local varieties were cross-pollinated with a plant population obtained by the cross-hybridization of two independent hybrids [GK3/H × MT and (A188 × A619) × BMSC] each of which was found to respond in anther culture. Ten kernels from the harvested ears were bulked, planted in the field and the plants selected for absence of undesirable agronomic traits (such as tillering, smut, lodging) were sibbed. After harvesting, the 30 best ears were kept and used for bulking an equal number of kernels per ear. These bulks represented the anther culture synthetic MR Syn A/85. One bulk of the synthetic was planted, and tassels were collected and processed in the described manner. From the cultured anthers a response of 0 to about 20 % of embryoid formation was observed per Petri dish. Out of more than 300 haploid embryoids derived from different tassels of the MS Syn A/85, about 20 % developed into regenerable embryogenic cultures after transfer from the initiation medium to haploid maintenance medium. From the regenerable cultures the H 229 culture was selected on the basis of its predominantly early-embryogenic callus phenotype, its fast propagation rate, its plant regeneration capacity after long-term surface and suspension culture (more than two years before using for further crossing cycle) and its ability to divide and to form colonies and to regenerate plants from protoplasts.

### Example 3:

### Production of the highly embryogenic culture DSM 6009.

Two previously selected genotypes developing predominantly early-embryogenic culture from embryos or anthers were cross-pollinated in order to combine the desirable characteristics of both. Female flowers of the regenerated H 229 plants, 30 months after initiation, were pollinated with the pollen from OK 281 plants regenerated 10 months after initiation. The obtained immature hybrid embryos were incubated on somatic culture initiation and maintenance medium as well as on plant regeneration medium. One of the embryos forming early-embryogenic culture on the plant regeneration medium, DSM 6009 (HE 89), was selected, maintained and used to initiate suspension cultures. Only two months after inoculation of the donor hybrid embryo (HL29 × OK281) the first successful protoplast culture was started, which led to the formation of fertile protoplast-derived plants. DSM 6009 has all the desirable features of its parents and is superior to them in many tissue culture traits listed earlier.

### Example 4:

### Evaluation of the suspension culture medium N6M for the retention of regenerability in the selected maize tissue cultures

Since the development of the suspension culture medium N6M 3 independently selected regenerable genotypes (including early- and late-embryogenic, somatic and haploid cultures) have been tested for more than two years in suspension culture for their regenerative and fertility properties. The 4C1 somatic mainly late-embryogenic culture, the C2-A somatic, late embryogenic culture, and the H 229 haploid mainly early-embryogenic culture were maintained in N6M suspension culture medium. Although suspension cultures show a faster propagation rate, the plant regeneration capacity of these selected genotypes was better when the embryoids were plated from suspension cultures than from infrequently passaged (about 2-month intervals) agar surface cultures. In all three cases, after two years of maintenance in N6M suspension culture fertile plants could be obtained. Similar observations were made with other cultures after one year of maintenance in this suspension culture. Therefore N6M suspension culture medium is preferred for maintenance of the cultures before protoplast isolation.

### Establishment and Maintenance of HE/89 Suspension Culture

The less than 1 mm embryos (H 229 × OK 281) obtained from a greenhouse-grown plant 12 days after pollination were incubated on N6M without 2,4-D for 3 weeks followed by two subcultures on N6M and MSG medium at 10-14-day intervals before the suspension culture is started in N6M medium. For regular maintenance 4 g of cells are used to start a new culture cycle of 5-7 days in 50 ml N6M medium in 100 ml Erlenmeyer flasks. All the cultures are grown under continuous or periodic illumination by fluorescent tubes at 22-25°C.

### Protoplast Isolation and Culture

2 g of fresh-weight cell material from suspension cultures 2 days after the last transfer was crushed with forceps in 1 ml washing solution (WS), to which 1 % of nPG stock solution was added. This treatment reduced the size of the 2 to 3 mm colonies. The crushed cell suspension was washed twice with nPG-WS. The washed cells are incubated in 10 ml protoplast isolation medium for 4 hours in the dark with or without gentle shaking. After enzyme treatment the solution is passed through a 210 micron and 60 micron screen followed by centrifugation at 1000 rpm. The pellet is resuspended in 10 ml of 0.6 M sucrose + nPG and 1 ml of WS + nPG is overlaid for the second centrifugation. The floated protoplasts are collected and sedimented again in 15 ml WS + nPG in the third centrifugation. The number of protoplasts is counted using a Buerker or Thoma chamber before the third centrifugation. The protoplasts are cultured in 2 ml of liquid medium or are embedded in 0.5-0.7 % low gel point agarose (Sigma) by mixing the protoplasts gently with 2 ml of a 1:1 double strength ppN6M/89 and 1.2 % melted agarose solution after a previous temperature adjustment to 45°C for both solutions. The protoplasts are cultured under dim light at 25°C. For feeding the protoplasts in the liquid medium 0.5 ml N6M medium is added after 3 weeks, followed by the addition of a further 1 ml 2-3 days later. Then after 3-4 days the whole culture was put into 10 ml fresh N6M medium and was shaken. The agarose-embedded cultures are placed either on solidified N6M with or without 2,4-D or into 10-50 ml liquid N6M medium after 2-4 weeks, in most cases after 3 weeks.

### Plant Regeneration and Growth

From the regenerated calli on agar medium 0.2-1 g of embryogenic callus is placed on 100 ml of N6M lacking 2,4-D in glass jars with a lid containing an aerating sponge plug or in plastic containers. This step is repeated until well-developed plants are obtained from the embryoids and the plantlets. The bigger plants (above 7 cm) are removed for transplantation into pots of different sizes containing various kinds of horticultural soils rich in humus. The plants with emerged silks and pollen-shedding tassels are self- or sib-pollinated.

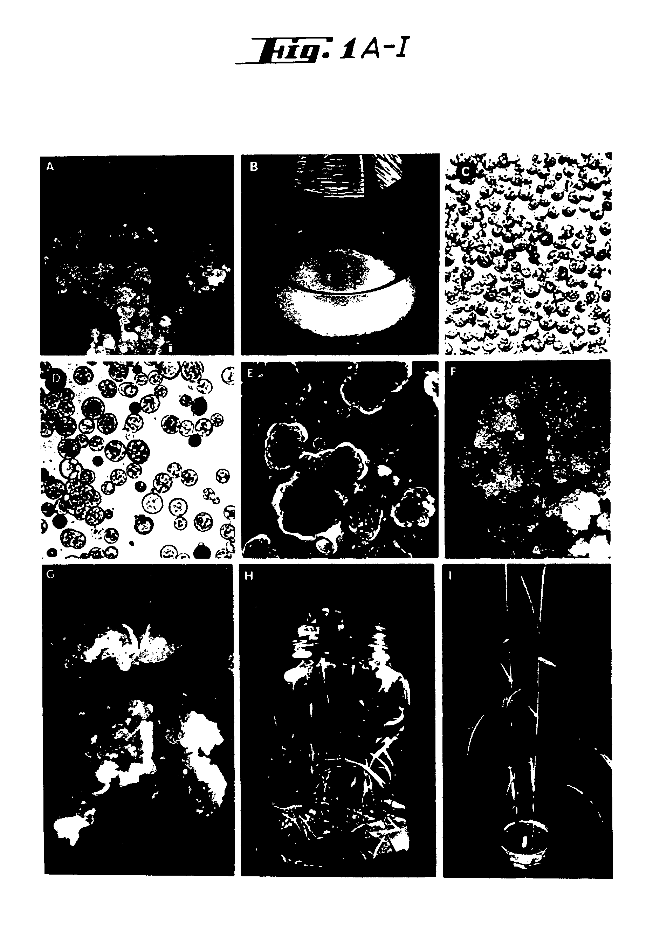

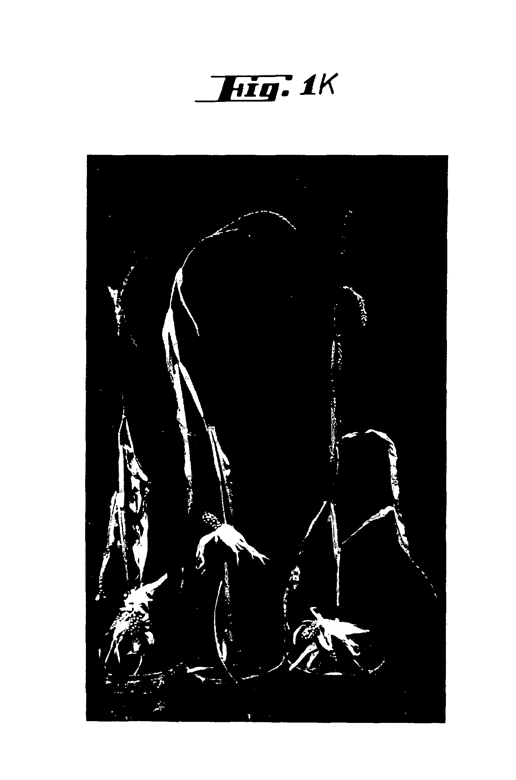

Figure 1 A-K: shows the various cell types and developmental stages involved in culturing DSM 6009, the DSM 6009 embryogenic maize culture and the regeneration of normal plants from it via the protoplast culture process. A: established early embryogenic culture, B: semifine suspension culture ready for protoplast isolation, C: protoplasts 6 hours after isolation, D: the first divisions 32 hours after isolation E: colonies 8 days after isolation, F, G: early and late embryogenic cultures from protoplasts after 2 months, respectively, regenerated plants from protoplasts before (H) and after (I) transplantation into soil, K: mature protoplast-derived plants with seed, 3 1/2 months after transfer into soil.

### Definitions

### H 229:

A regenerable haploid Zea mays (L.) tissue culture and genotype which was obtained via anther culture from MS Syn A/85 anther culture synthetic and which was produced by successive cross- and sib-pollinations with the inclusion of the following genotypes: A188, A629, GK 3/H (inbreds), Black Mexican Sweet Corn (BMSC), Mangelsdorf's Tester (MT) (genetic stocks), white dent local variety (WDLV) and Golden Glow (GG) (local varieties). The H 229 culture is predominantly early-embryogenic, and readily forms suspension cultures, while the suspension culture protoplasts develop into plants under defined conditions. The H 229 plants obtained from the H 229 tissue culture are female-fertile, giving seeds upon pollination with viable pollen grains.

### OK 281:

A regenerable, predominantly early-embryogenic, somatic Zea mays (L.) tissue culture and genotype obtained via immature embryo culture from the MR Syn So₂o₂/87 which was produced by successive cross-, sib- and self-pollinations with the inclusion of the following genotypes: A 188, GK 3/H, W 64 A, Oh 43o₂o₂ (inbreds), MT (genetic stock), white flint local variety (WFLV) (local variety) and H Temp Ao₂o₂ (o₂o₂ synthetic).

The OK 281 plants obtained from the OK 281 cultures produce seeds via self-, sib- or cross-pollination under appropriate conditions. The OK 281 genotype is homozygous for the recessive opaque gene (o₂o₂).

### DSM 6009 (HE/89):

A regenerable, highly embryogenic, predominantly early-embryogenic somatic Zea mays (L.) culture and genotype obtained via immature embryo culture following cross-pollination between the two previously selected genotypes: H 229 and OK 281. The H 229 and OK 281 genotypes were maintained for 30 and 10 months, respectively, in culture before plants were regenerated for cross-pollinations. The DSM 6009 genotype is especially adapted to in vitro culture requirements as a consequence of its descent from ancestors which were selected for their tissue culture characteristics. Thus, DSM 6009 possesses a unique combination of the following traits:
1. Formation of hormone autotrophic embryogenic callus which grows well on standard plant tissue culture media.
2. Reproducible formation of callus-like early embryogenic (type II callus) cultures from mature somatic embryos on auxin-containing media.
3. Reliable long-term plant regeneration capability.
4. Reproducible conversion of callus-like early-embryogenic (type II callus) cultures into suspension cultures.
5. Abundant protoplast release one month after transfer of the callus into liquid culture.
6. High frequency of regeneration of fully fertile plants from callus, suspensions and protoplasts.

### Early- and late-embryogenic cultures:

A special type of maize (Zea mays L.) tissue cultures in which a rapid propagation rate of early stages of somatic embryos is observed. These embryoids do not mature into clearly visible embryoids which are characterized by their size, smooth, scutellum-like structures bearing leaf and root primordia. In contrast, the early-embryogenic cultures have the appearance of a homogeneous callus culture. This culture type is referred to as type II or friable embryogenic callus culture in the relevant literature. The early-embryogenic cultures develop into late-embryogenic cultures before plant regeneration. In the late-embryogenic cultures the embryoids are enlarged with well-developed scutellum-like bodies, green spots or leaf and shoot primordia, as well as with root primordia or small roots. In DSM 6009 cultures both callus types are easily accessible and interchangeable depending on the culture conditions.

### Somatic culture initiation and maintenance medium

The modified MS medium (Murashige, T. and Skoog, F. Physiol. Plant., 15 (1962) 473-497) as published by Green, C.E. and Phillips R.L. Crop Sci., 15 (1975) 417-421 but with the following modifications and additions (final concentration in mg/l): Bacto tryptone 500 mg, L-asparagine 150 mg.

### Anther culture initiation medium

N6 macro-elements, glycine and vitamins as in Chu et al., Scientia Sinica, 18 (1975) 659, MS micro-elements, EDTA and FeSO₄ · 7H₂O as in Murashige, T., and Skoog, F., Physiol. Plant, 15 (1962) 473, and the following components (per l) : L-proline 100 mg, Bacto tryptone (BT) 600 mg, sucrose 120 g, benzyl-adenine 1 mg, naphthylacetic acid 1 mg, 2,4-dichlorophenoxyacetic acid (2,4-D) 2 mg, activated charcoal 5 g. The pH is adjusted to 5.8 ± 0.05 with KOH before autoclaving and addition of activated charcoal. The medium is solidified with 5 g/l agarose.

### Haploid culture maintenance medium

Macro-elements, micro-elements, EDTA, FeSO₄ · 7H₂O, amino acids and vitamins as in the anther culture initiation medium. The altered components are (per l): 2,4-D 0.5 mg, sucrose 30 g, agar 8 g. The pH is adjusted to 5.8 ± 0.05 before autoclaving.

### Suspension culture (N₆M)-medium

The same as the haploid culture maintenance medium without agar.

### Protoplast culture medium (ppN₆M)-medium

A modified N₆M-medium with the following additional ingredients or altered ingredients (final concentrations per l).

MgSO₄ · 7H₂O : 370 mg; CaCl₂ · 2H₂O : 300 mg; sucrose 1 g; glucose 50 g; fructose 30 g; maltose 2.5 g; galactose 2.5 g; galacturonic acid 500 mg; glucuronic acid 500 mg; L-asparagine 500 mg; L-glutamine 100 mg, L-serine 100 mg; 2,4-dichlorophenoxyacetic acid 0.4 g; naphthylacetic acid 0.7 mg and zeatin (mixed isomers) 0.7 mg. The pH, without adjustment, is in the range of 4.5-5.0.

### Protoplast isolation medium (cellulytic solution)

10 ml of the protoplast isolation solution are prepared from the following filter-sterilized (fs) or autoclaved (a) stock solutions:

| | |
|---|---|
| 0.3-0.5 ml | desalted cellulase/pectinase stock (fs) |
| 2.0 ml | washing solution (fs) |
| 4.2-4.4 ml | double-dist. H₂O (a) |
| 0.5 ml | BSA stock solution (fs) |
| 0.1 ml | n-PG solution (fs) |
| 0.1 ml | 1 M CaCl₂ solution (a) |
| 0.1 ml | 1 M MgSO₄ solution (a) |
| 2.5 ml | osmotic solution (fs) |

### Desalted cellulase/pectinase stock solution

10 g Cellulase Onozuka RS and 1 g Pectolyase Y23 are dissolved in double-distilled H₂O at 4°C. Unsoluble debris is removed by centrifugation (6000 rpm for 10 min). The supernatant is desalted by running through a Sephadex G25 column (column volume 200-250 ml). The salt-free enzyme-containing fractions are pooled and adjusted to a final volume of 50 ml. They are filter-sterilized through 0.22 µm membrane filters and then deep frozen in 0.5 ml aliquots.

### Washing solution (WS)

N₆M medium without Fe, EDTA, 2.4-D and (NH₄)₂SO₄. It contains the following sugars: sucrose 10 g/l, glucose 55 g/l and fructose 55 g/l.

### BSA - Stock solution

From lyophilized bovine serum albumin, fraction V (Merck), a stock solution is prepared, containing 40 mg BSA in 1 ml double-distilled H₂O. Aliquots are kept deep frozen prior to use.

### nPG - solution

50 mg n-propyl gallate are dissolved in 2 ml ethanol. 8 ml double-distilled H₂O are added and the solution is filter-sterilized. 1% freshly prepared nPG solution is added to the washing solution (WS-nPG) and 0.6 M sucrose solution (0.6 Su + nPG).

### Osmotic solution

100 ml contain KNO₃ 4.04 g; KH₂PO₄ 2.72 g; K₂HPO₄ 0.94 g; fructose 7.2 g; glucose 8.0 g; L-proline 0.68 g. The solution is filter-sterilized.

## Claims

1. A genotype of Zea mays (L.) which is auxin-autotrophic and from which protoplasts, which reproducibly and stably regenerate into normal fertile plants, can be produced, as well as the regenerated plants and parts and progeny of those plants.

2. An embryogenic cell culture produced from the genotype as claimed in claim 1.

3. The embryogenic cell culture DSM 6009.

4. Protoplasts produced from the genotype as claimed in claim 1, as well as cells regenerated from these protoplasts, said cells having the capability to regenerate fertile plants.

## Patentansprüche

1. Ein auxin-autotropher Genotyp von Zea mays (L) aus dem Protoplasten isoliert werden können, die in einer stabielen und reproduzierbaren Weise zu normalen fruchtbaren Pflanzen regenerieren, sowie auch die regenerierten Pflanzen und Pflanzenteile und Nachkommen dieser Pflanzen.

2. Eine embryogene Zellkultur aus dem Genotyp gemäß Anspruch 1 produziert.

3. Die embryogene Zellkultur DSM 6009.

4. Protoplasten aus dem Genotyp gemäß Anspruch 1 produziert, sowie auch aus diesen Protoplasten regenerierte Zellen, wobei genannte Zellen die Kapazität haben zu fruchtbaren Pflanzen regeneriert zu werden.

## Revendications

1. Un génotype autotrophe en auxine de Zea mays (L.) a partir duquel on peut obtenir des protoplastes, lesquels protoplastes se régénèrent en plantes normales fertiles d'une façon réproductible et stable, ainsi que les plantes régénérées et des parties et la progéniture de cettes plantes.

2. Une culture cellulaire embryogénique, produite du génotype selon la revendication 1.

3. La culture cellulaire embryogénique DSM 6009.

4. Des protoplastes produits à partir du génotype selon la revendication 1, ainsi que des cellules régénérées de ces protoplastes, les-dites cellules ayant la capacité de régénérer des plantes fertiles.
